# EUROPEAN PATENT APPLICATION

(11) **EP 4 088 644 A1**
(43) Date of publication of application: **16.11.2022**
(21) Application number: 20912245.6
(22) Date of filing: 25.11.2020
(51) Int. Cl.: A61B 1/045

(54) **ENDOSCOPE SYSTEM AND OPERATION METHOD THEREOF**

(30) Priority: 09.01.2020 JP 2020002216
(71) Applicant: FUJIFILM Corporation, Tokyo 106-8620 (JP)
(72) Inventor: KUBO, Masahiro, Ashigarakami-gun, Kanagawa 258-8538 (JP)
(74) Representative: Hughes, Andrea Michelle
(86) International application number: PCT/JP2020/043834
(87) International publication number: WO 2021/140766

(57) **Abstract**

There are provided an endoscope system and a method of operating the endoscope system that can switch light emission to mono-light emission and multi-light emission without being cumbersome and stressful for a user and without causing a user to be conscious (recognize).

Mono-light emission where only specific illumination light is emitted and multi-light emission where first illumination light and second illumination light having emission spectra different from each other are emitted while being switched according to a specific light emission pattern are automatically switched on the basis of the activation or non-activation of an analysis processing mode, the activation or non-activation of a multi-image display mode, and a predetermined light source switching condition.

## Description

### BACKGROUND OF THE INVENTION

### 1. Field of the Invention

The present invention relates to an endoscope system and a method of operating the endoscope system that illuminate a subject with a plurality of types of illumination light having different wavelength ranges while switching the plurality of types of illumination light and display observation images corresponding to the respective types of illumination light while switching the observation images.

### 2. Description of the Related Art

In recent years, an endoscope system comprising a light source device, an endoscope, and a processor device has been widely used in a medical field. In the endoscope system, an object to be observed is irradiated with illumination light from an endoscope, and the image of the object to be observed is displayed on a display on the basis of RGB image signals that are obtained in a case where the image of the object to be observed, which is being illuminated with the illumination light, is picked up by an image pickup element of the endoscope.

Further, in recent years, an object to be observed has been illuminated with a plurality of types of illumination light having different wavelength ranges to obtain much diagnosis information from the object to be observed. For example, in WO2019/093356A, multi-light emission where first illumination light and second illumination light are emitted while being switched according to a specific light emission pattern is performed, and a first observation image and a second observation image are displayed on a display while being switched according to a specific display pattern.

### SUMMARY OF THE INVENTION

In recent years, in the case of multi-light emission of the first illumination light and the second illumination light, analysis processing, such as artificial intelligence (AI) has been performed on second image signals obtained from the image pickup of an object to be observed illuminated with the second illumination light for analysis processing that is one illumination light. Further, the analysis result of the analysis processing is superimposed and displayed on a display image based on first image signals obtained from the image pickup of the object to be observed illuminated with the first illumination light, so that an analysis processing mode where a display image with an analysis result is displayed on a display is also performed.

In a case where a multi-image display mode where the first observation image and the second observation image are displayed on the display while being switched according to the specific display pattern is performed as disclosed in WO2019/093356A in addition to the analysis processing mode, mono-light emission where only specific illumination light is emitted and the multi-light emission have been required to be switched without being cumbersome and stressful for a user and without causing a user to be conscious (recognize).

An object of the present invention is to provide an endoscope system and a method of operating the endoscope system that can switch light emission to mono-light emission and multi-light emission without being cumbersome and stressful for a user and without causing a user to be conscious (recognize).

An endoscope system according to an aspect of the present invention comprises: a plurality of semiconductor light sources that emit light having wavelength ranges different from each other; a light source processor that controls the plurality of semiconductor light sources and performs a control related to mono-light emission where only specific illumination light is emitted and a control related to multi-light emission where a plurality of types of illumination light, which include first illumination light and second illumination light having emission spectra different from each other, are emitted while being switched according to a specific light emission pattern; an image pickup sensor that outputs image signals including a first image signal obtained from image pickup of an object to be observed illuminated with the first illumination light and a second image signal obtained from image pickup of the object to be observed illuminated with the second illumination light in a case of the multi-light emission; and an image control processor. The image control processor is capable of activating at least an analysis processing mode where a display image with an analysis result in which an analysis result obtained from analysis processing based on the second image signal is displayed on a display image based on the first image signal is displayed on a display; is capable of activating a mono-image display mode where a control to display a specific observation image, which is obtained from image pickup of the object to be observed illuminated with the specific illumination light, on the display is performed; and is capable of activating a multi-image display mode where a control to display a plurality of observation images, which include a first observation image based on the first image signal and a second observation image based on the second image signal, on the display while switching the plurality of observation images according to a specific display pattern is performed. The image control processor automatically switches the mono-light emission and the multi-light emission on the basis of activation or non-activation of the analysis processing mode, activation or non-activation of the multi-image display mode, and a predetermined light source switching condition.

It is preferable that, in a case where the analysis processing mode is not activated and the multi-image display mode is activated, the image control processor automatically switches light emission to the multi-light emission from the mono-light emission during activation of the mono-light emission. It is preferable that, in a case where light emission is automatically switched to the mono-light emission from the multi-light emission and a mode is automatically switched to the mono-image display mode from the multi-image display mode since a multi-image display allowable condition included in the light source switching condition is not satisfied, the image control processor automatically switches light emission to the multi-light emission from the mono-light emission and activates the multi-image display mode in a case where a multi-image display restart condition is satisfied and a restart allowable condition is satisfied in the light source switching condition, and prohibits light emission from being automatically switched to the multi-light emission in a case where the multi-image display restart condition is satisfied and the restart allowable condition is not satisfied.

It is preferable that the restart allowable condition is a case where a user does not select the mono-light emission. It is preferable that the restart allowable condition is a case where a restart allowable time does not elapse yet after light emission is switched to the mono-light emission. It is preferable that the restart allowable condition is a case where the multi-light emission is implementable. It is preferable that the endoscope system further comprises a magnification change unit that is used to change a magnification of the object to be observed, and the restart allowable condition is a case where use or non-use of the magnification change unit is not switched. It is preferable that the restart allowable condition is capable of being set by a user. It is preferable that the multi-image display restart condition is that a change in an image pickup condition about the object to be observed is within a multi-light emission allowable range.

It is preferable that, in a case where the analysis processing mode is activated and the multi-image display mode is not activated, the image control processor automatically switches light emission to the multi-light emission from the mono-light emission according to start of the activation of the analysis processing mode during activation of the mono-light emission. It is preferable that, in a case where light emission is automatically switched to the mono-light emission from the multi-light emission and a mode is automatically switched to the mono-image display mode from the analysis processing mode since an analysis processing allowable condition included in the light source switching condition is not satisfied, the image control processor automatically switches light emission to the multi-light emission from the mono-light emission and activates the analysis processing mode in a case where an analysis processing restart condition is satisfied and a restart allowable condition is satisfied in the light source switching condition, and prohibits light emission from being automatically switched to the multi-light emission in a case where the analysis processing restart condition is satisfied and the restart allowable condition is not satisfied.

It is preferable that, in a case where the analysis processing mode is activated and the multi-image display mode is activated, the image control processor automatically switches light emission to the multi-light emission from the mono-light emission according to start of the activation of the analysis processing mode and the multi-image display mode during activation of the mono-light emission, prohibits light emission from being automatically switched to the mono-light emission in a case where any of an analysis processing allowable condition and a multi-image display allowable condition included in the light source switching condition is satisfied during activation of the multi-light emission, and automatically switches light emission to the mono-light emission from the multi-light emission in a case where the analysis processing allowable condition is not satisfied and the multi-image display allowable condition is not satisfied during activation of the multi-light emission.

According to another aspect of the present invention, there is provided a method of operating an endoscope system including: a plurality of semiconductor light sources that emit light having wavelength ranges different from each other; a light source processor that controls the plurality of semiconductor light sources and performs a control related to mono-light emission where only specific illumination light is emitted and a control related to multi-light emission where a plurality of types of illumination light, which include first illumination light and second illumination light having emission spectra different from each other, are emitted while being switched according to a specific light emission pattern; an image pickup sensor that outputs a first image signal obtained from image pickup of an object to be observed illuminated with the first illumination light and a second image signal obtained from image pickup of the object to be observed illuminated with the second illumination light in a case of the multi-light emission; and an image control processor. The image control processor is capable of activating an analysis processing mode where a display image with an analysis result in which an analysis result obtained from analysis processing based on the second image signal is displayed on a display image based on the first image signal is displayed on a display; is capable of activating a mono-image display mode where a control to display a specific observation image, which is obtained from image pickup of the object to be observed illuminated with the specific illumination light, on the display is performed; and is capable of activating a multi-image display mode where a control to display a plurality of observation images, which include a first observation image based on the first image signal and a second observation image based on the second image signal, on the display while switching the plurality of observation images according to a specific display pattern is performed. Further, the image control processor automatically switches the mono-light emission and the multi-light emission on the basis of activation or non-activation of the analysis processing mode, activation or non-activation of the multi-image display mode, and a predetermined light source switching condition.

According to the present invention, it is possible to switch light emission to mono-light emission and multi-light emission without being cumbersome and stressful for a user and without causing a user to be conscious (recognize).

### BRIEF DESCRIPTION OF THE DRAWINGS

Fig. 1 is a diagram showing the appearance of an endoscope system.
Fig. 2 is a block diagram showing the functions of the endoscope system.
Fig. 3 is a graph showing the emission spectra of violet light V, blue light B, green light Q and red light R.
Fig. 4 is a diagram illustrating a specific light emission pattern that is used in an analysis processing mode.
Fig. 5 is a diagram illustrating a specific light emission pattern that is used in a multi-image display mode.
Fig. 6 is a diagram illustrating image display in the analysis processing mode.
Fig. 7 is a diagram illustrating image display in the multi-image display mode.
Fig. 8 is a diagram illustrating that a mode is automatically switched to a mono-image display mode from the multi-image display mode in a switching pattern A.
Fig. 9 is a diagram illustrating that a mode is automatically switched to the mono-image display mode from the multi-image display mode in a case where the use time of multi-light emission is equal to or longer than a time threshold value.
Fig. 10 is a diagram illustrating that a mode is automatically switched to the mono-image display mode from the multi-image display mode in a case where the number of times of storage of a static image is equal to or larger than a number-of-times threshold value.
Fig. 11 is a block diagram showing the functions of an image-pickup-condition acquisition unit.
Fig. 12 is a diagram illustrating that a mode is automatically switched to the mono-image display mode from the multi-image display mode in a case where an observation portion is changed to a second portion from a first portion or in a case where an observation portion is changed to the first portion from the second portion.
Fig. 13 is a diagram illustrating that a mode is automatically switched to the mono-image display mode from the multi-image display mode in a case where brightness is equal to or lower than a first brightness threshold value or brightness is equal to or higher than a second brightness threshold value.
Fig. 14 is a diagram illustrating that a mode is automatically switched to the mono-image display mode from the multi-image display mode in a case where the variation of magnification exceeds a magnification threshold value.
Fig. 15 is a diagram illustrating that a mode is automatically switched to the mono-image display mode from the multi-image display mode in a case where the variation of an observation distance exceeds a distance threshold value.
Fig. 16 is a diagram illustrating that a mode is automatically switched to the mono-image display mode from the multi-image display mode in a case where a shake amount exceeds a shake-amount threshold value.
Fig. 17 is an image diagram showing a multi-image display allowable condition-setting menu.
Fig. 18 is a diagram illustrating that a mode is automatically switched to the multi-image display mode from the mono-image display mode in the switching pattern A.
Fig. 19 is a diagram illustrating that a mode is automatically switched to the multi-image display mode from the mono-image display mode in a case where an observation portion returns to the original observation portion and the original observation portion is observed.
Fig. 20 is a diagram illustrating that a mode is automatically switched to the multi-image display mode from the mono-image display mode in a case where brightness is equal to or higher than the first brightness threshold value or brightness is equal to or lower than the second brightness threshold value.
Fig. 21 is a diagram illustrating that a mode is automatically switched to the multi-image display mode from the mono-image display mode in a case where the variation of magnification is less than the magnification threshold value.
Fig. 22 is a diagram illustrating that a mode is automatically switched to the multi-image display mode from the mono-image display mode in a case where the variation of an observation distance is equal to or smaller than the distance threshold value.
Fig. 23 is a diagram illustrating that a mode is automatically switched to the multi-image display mode from the mono-image display mode in a case where a shake amount is equal to or smaller than the shake-amount threshold value.
Fig. 24 is a diagram illustrating that a mode is automatically switched to the mono-image display mode from the analysis processing mode in a switching pattern B.
Fig. 25 is a diagram illustrating that a mode is automatically switched to the analysis processing mode in a switching pattern C.
Fig. 26 is a diagram illustrating that a mode is automatically switched to the multi-image display mode in the switching pattern C.
Fig. 27 is a diagram illustrating that a mode is automatically switched to the mono-image display mode in the switching pattern C.
Fig. 28 is a flowchart showing a series of flows in the switching pattern A.
Fig. 29 is a flowchart showing a series of flows in the switching pattern B.
Fig. 30 is a flowchart showing a series of flows in the switching pattern C.

### DESCRIPTION OF THE PREFERRED EMBODIMENTS

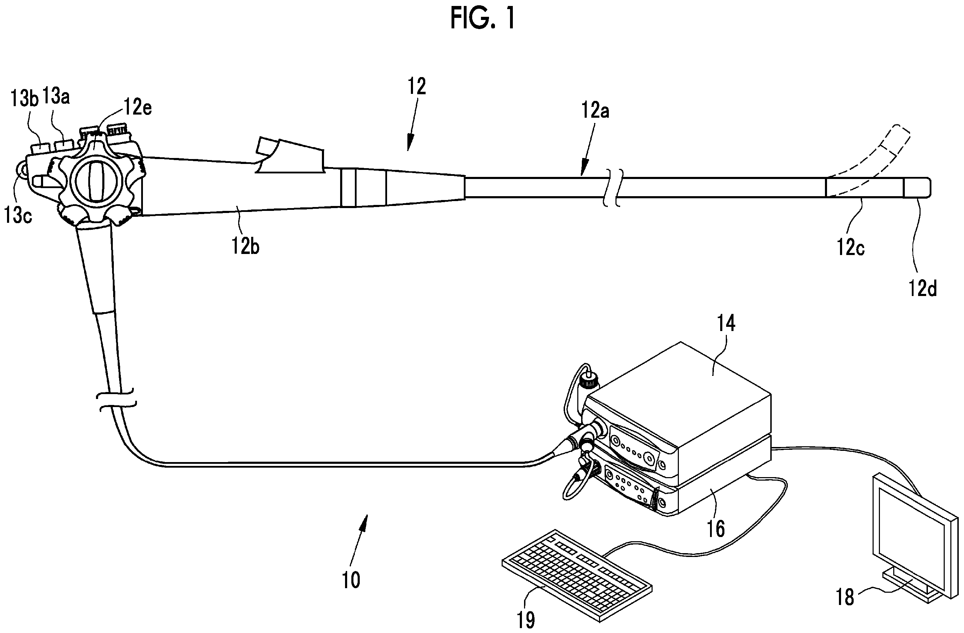

As shown in Fig. 1, an endoscope system 10 includes an endoscope 12, a light source device 14, a processor device 16, a display 18, and a user interface 19. The endoscope 12 is optically connected to the light source device 14, and is electrically connected to the processor device 16. The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. In a case where angle knobs 12e of the operation part 12b are operated, the bendable part 12c is operated to be bent. As the bendable part 12c is operated to be bent, the distal end part 12d faces in a desired direction. The user interface 19 includes a mouse and the like in addition to a keyboard shown in Fig. 1.

Further, the operation part 12b is provided with a mode changeover SW 13a, a static-image-acquisition instruction part 13b, and a zoom operation part 13c in addition to the angle knobs 12e. The mode changeover SW 13a is used to switch a mono-image display mode, an analysis processing mode, and a multi-image display mode. In the mono-image display mode, only normal light (specific illumination light) is emitted (mono-light emission) and a normal image (specific observation image) is displayed on the display 18. In the mono-image display mode, any of first illumination light or second illumination light may be emitted instead of the normal light. Further, both the analysis processing mode and the multi-image display mode can be activated at the same time.

In the analysis processing mode, first illumination light and second illumination light having emission spectra different from each other are emitted while being switched according to a specific light emission pattern (multi-light emission). Further, in the analysis processing mode, processing for forming a display image to be displayed on the display 18 is performed on an image based on the first illumination light but analysis processing, such as artificial intelligence (AI) processing, is performed on an image based on the second illumination light. The result of the analysis processing is superimposed and displayed on the display image.

In the multi-image display mode, the first illumination light and the second illumination light having emission spectra different from each other are emitted while being switched according to a specific light emission pattern (multi-light emission). Further, in the multi-image display mode, a first observation image based on first image signals obtained in a case where an object to be observed is illuminated with the first illumination light and a second observation image based on second image signals obtained in a case where the object to be observed is illuminated with the second illumination light are displayed on the display 18 while being switched according to a specific display pattern.

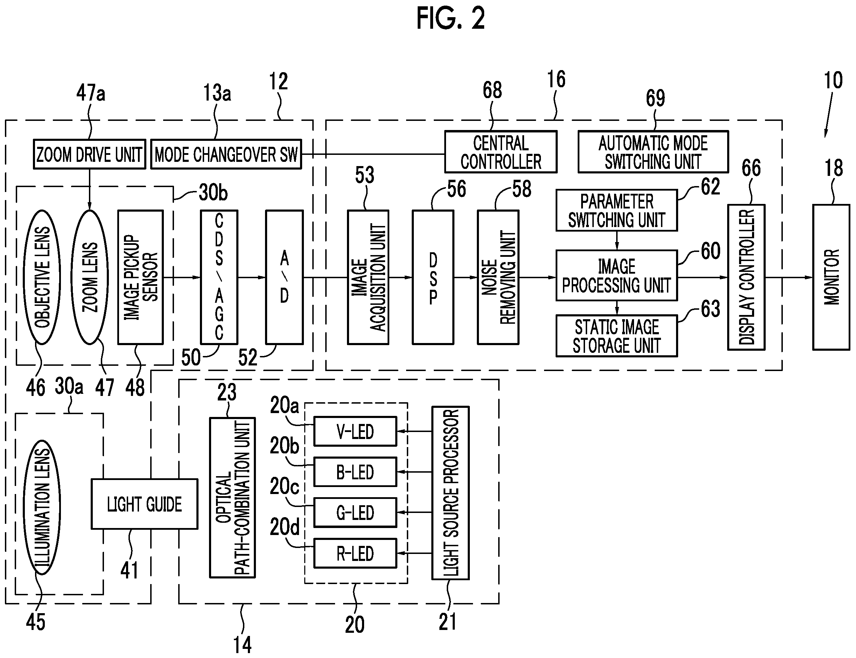

The static-image-acquisition instruction part 13b is used to instruct a static image storage unit 63 (see Fig. 2) to store the static image of the object to be observed. The zoom operation part 13c is used to operate a zoom lens 47 and a zoom drive unit 47a (see Fig. 2) that are provided in the endoscope 12.

The processor device 16 is electrically connected to the display 18 and the user interface 19. The display 18 outputs and displays image information and the like. The user interface 19 has a function to receive an input operation, such as function settings. An external recording unit (not shown), which records image information and the like, may be connected to the processor device 16.

As shown in Fig. 2, the light source device 14 includes a light source unit 20, a light source processor 21, and an optical path-combination unit 23. The light source unit 20 can emit light having a plurality of wavelength ranges, and can change the light emission ratio of the light having each wavelength range. In this specification, "light having a plurality of wavelength ranges different from each other" means that the plurality of wavelength ranges may partially overlap with each other without meaning that the plurality of wavelength ranges do not overlap with each other at all. The light source unit 20 includes a violet light emitting diode (V-LED) 20a, a blue light emitting diode (B-LED) 20b, a green light emitting diode (G-LED) 20c, and a red light emitting diode (R-LED) 20d in order to emit light having a plurality of wavelength ranges. Since the light source unit 20 may be provided with a plurality of semiconductor light sources, a laser diode (LD) may be used instead of the LED.

The light source processor 21 controls the drive of the LEDs 20a to 20d. The optical path-combination unit 23 combines the optical paths of four types of color light that are emitted from the four color LEDs 20a to 20d. The inside of an object to be examined is irradiated with the pieces of light, which are combined by the optical path-combination unit 23, through a light guide 41 inserted into the insertion part 12a and an illumination lens 45.

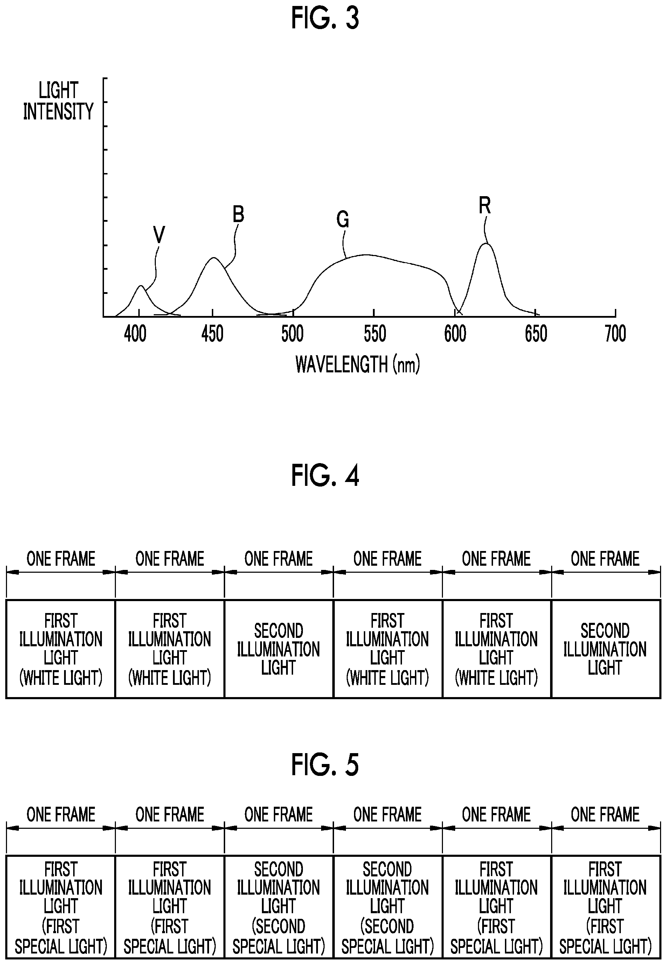

As shown in Fig. 3, the V-LED 20a generates violet light V of which the central wavelength is in a range of 405±10 nm and the wavelength range is in a range of 380 to 420 nm. The B-LED 20b generates blue light B of which the central wavelength is in a range of 450±10 nm and the wavelength range is in a range of 420 to 500 nm. The G-LED 20c generates green light G of which the wavelength range is in a range of 480 to 600 nm. The R-LED 20d generates red light R of which the central wavelength is in a range of 620 to 630 nm and the wavelength range is in a range of 600 to 650 nm.

The light source processor 21 controls the V-LED 20a, the B-LED 20b, the G-LED 20c, and the R-LED 20d. Since the light source processor 21 independently controls each of the LEDs 20a to 20d, each of violet light V, blue light B, green light G, and red light R can be emitted independently while the amount of light is changed. Further, the light source processor 21 controls the respective LEDs 20a to 20d so that white light of which a light amount ratio between violet light V, blue light B, green light G, and red light R is Vc:Bc:Gc:Rc is emitted in the mono-image display mode. Vc, Bc, Gc, and Rc are larger than 0.

In this specification, the light amount ratio includes a case where the ratio of at least one semiconductor light source is 0 (zero). Accordingly, the light amount ratio includes a case where any one or two or more of the respective semiconductor light sources are not turned on. For example, even though only one semiconductor light source is turned on and the other three semiconductor light sources are not turned on as in a case where a light amount ratio between violet light V, blue light B, green light G, and red light R is 1:0:0:0, it is regarded that the light source unit 20 has a light amount ratio.

Further, in a case where the first illumination light and the second illumination light are emitted in the multi-image display mode or the analysis processing mode while being automatically switched, the light source processor 21 allows the first illumination light to be emitted according to a first light emission pattern and allows the second illumination light to be emitted according to a second light emission pattern as the specific light emission pattern. Specifically, it is preferable that the first light emission pattern is any of a first A light emission pattern where the number of frames of a first illumination period in which the first illumination light is emitted is the same in each first illumination period or a first B light emission pattern where the number of frames of the first illumination period is different in each first illumination period.

It is preferable that the second light emission pattern is any of a second A pattern where the number of frames of a second illumination period in which the second illumination light is emitted is the same in each second illumination period and the emission spectrum of the second illumination light is the same in each second illumination period, a second B pattern where the number of frames of the second illumination period is the same in each second illumination period and the emission spectrum of the second illumination light is different in each second illumination period, a second C pattern where the number of frames of the second illumination period is different in each second illumination period and the emission spectrum of the second illumination light is the same in each second illumination period, or a second D pattern where the number of frames of the second illumination period is different in each second illumination period and the emission spectrum of the second illumination light is different in each second illumination period. The emission spectrum of the first illumination light may be the same or different in each first illumination period.

In a case where the first light emission pattern is set to the first A pattern and the second light emission pattern is set to the second A pattern (the number of frames of the second illumination period is the same and the emission spectrum of the second illumination light is the same) as the specific light emission pattern used in the analysis processing mode, the first illumination period is set to two frames and the second illumination period is set to one frame as shown in Fig. 4. Since the first illumination light is used to generate a display image to be displayed on the display 18, it is preferable that a bright image is obtained in a case where the object to be observed is illuminated with the first illumination light. For example, it is preferable that the first illumination light is white light. On the other hand, since the second illumination light is used for analysis processing, it is preferable that an image suitable for analysis processing is obtained in a case where the object to be observed is illuminated with the second illumination light. For example, in a case where analysis processing is performed on the basis of shape information about superficial blood vessels, it is preferable that violet light V is used as the second illumination light. With regard to the specific light emission pattern, it is preferable that the first illumination period is set to be longer than the second illumination period and it is preferable that the first illumination period is set to two or more frames.

In a case where the first light emission pattern is set to the first A pattern and the second light emission pattern is set to the second A pattern (the number of frames of the second illumination period is the same and the emission spectrum of the second illumination light is the same) as the specific light emission pattern used in the multi-image display mode, the first illumination period is set to two frames and the second illumination period is set to two frames as shown in Fig. 5. In a case where the first observation image in which superficial blood vessels are enhanced and the second observation image in which medium-deep blood vessels present at a position deeper than the superficial blood vessels are enhanced are displayed on the display 18 in the multi-image display mode while being automatically switched, it is preferable that first special light of which the amount of violet light V is larger than the amount of the other color light (blue light B, green light G, and red light R) is used as the first illumination light and second special light of which the amount of green light G is larger than the amount of the other color light (violet light V, blue light G, and red light R) is used as the second illumination light.

As shown in Fig. 2, the light guide 41 is built in the endoscope 12 and a universal cord (a cord connecting the endoscope 12 to the light source device 14 and the processor device 16), and transmits the pieces of light, which are combined by the optical path-combination unit 23, to the distal end part 12d of the endoscope 12. A multimode fiber can be used as the light guide 41. For example, a thin fiber cable of which a total diameter of a core diameter of 105 µm, a cladding diameter of 125 µm, and a protective layer forming a covering is in a range of ϕ 0.3 to 0.5 mm can be used.

The distal end part 12d of the endoscope 12 is provided with an illumination optical system 30a and an image pickup optical system 30b. The illumination optical system 30a includes an illumination lens 45, and an object to be observed is irradiated with light transmitted from the light guide 41 through the illumination lens 45. The image pickup optical system 30b includes an objective lens 46, a zoom lens 47, and an image pickup sensor 48. Light reflected from the object to be observed is incident on the image pickup sensor 48 through the objective lens 46 and the zoom lens 47. Accordingly, the reflected image of the object to be observed is formed on the image pickup sensor 48. The zoom lens 47 can be moved along an optical axis by the zoom drive unit 47a. The size of the image of the object to be observed is increased or reduced depending on the movement of the zoom lens 47. A "magnification change unit" of the present invention corresponds to a configuration that includes the zoom operation part 13c, the zoom lens 47, and the zoom drive unit 47a.

The image pickup sensor 48 is a color image pickup sensor, and picks up the reflected image of an object to be examined and outputs image signals. It is preferable that the image pickup sensor 48 is a charge coupled device (CCD) image pickup sensor, a complementary metal-oxide semiconductor (CMOS) image pickup sensor, or the like. The image pickup sensor 48 used in the present invention is a color image pickup sensor that is used to obtain RGB image signals corresponding to three colors of R (red), G (green), and B (blue), that is, a so-called RGB image pickup sensor that comprises R-pixels provided with R-filters, G-pixels provided with G-filters, and B-pixels provided with B-filters.

In the case of the mono-image display mode, the image pickup sensor 48 outputs normal image signals that are obtained from the image pickup of the object to be observed illuminated with the normal light. In the case of the analysis processing mode or the multi-image display mode, the image pickup sensor 48 outputs first image signals that are obtained from the image pickup of the object to be observed illuminated with the first illumination light and second image signals that are obtained from the image pickup of the object to be observed illuminated with the second illumination light.

The image pickup sensor 48 may be a so-called complementary color image pickup sensor, which comprises complementary color filters corresponding to C (cyan), M (magenta), Y (yellow), and G (green), instead of an RGB color image pickup sensor. In a case where a complementary color image pickup sensor is used, image signals corresponding to four colors of C, M, Y, and G are output. Accordingly, the image signals corresponding to four colors of C, M, Y, and G need to be converted into image signals corresponding to three colors of R, G, and B by complementary color-primary color conversion. Further, the image pickup sensor 48 may be a monochrome image pickup sensor that includes no color filter. In this case, the light source processor 21 needs to cause blue light B, green light G, and red light R to be emitted in a time-sharing manner, and add demosaicing to the processing of image pickup signals.

The image signals output from the image pickup sensor 48 are transmitted to a CDS/AGC circuit 50. The CDS/AGC circuit 50 performs correlated double sampling (CDS) or auto gain control (AGC) on the image signals that are analog signals. The image signals, which have been transmitted through the CDS/AGC circuit 50, are converted into digital image signals by an analog/digital converter (A/D converter) 52. The digital image signals, which have been subjected to A/D conversion, are input to the processor device 16.

In the processor device 16, a program related to processing, such as automatic mode switching is stored in a program memory (not shown). In the processor device 16, the program stored in the program memory is executed by a central controller 68 formed of an image control processor, so that the functions of an image acquisition unit 53, a digital signal processor (DSP) 56, a noise removing unit 58, an image processing unit 60, a parameter switching unit 62, a display controller 66, and an automatic mode switching unit 69 are realized.

Digital color image signals output from the endoscope 12, such as the normal image signals, the first image signals, and the second image signals, are input to the image acquisition unit 53. The color image signals are RGB image signals that are formed of R-image signals output from the R-pixels of the image pickup sensor 48, G-image signals output from the G-pixels of the image pickup sensor 48, and B-image signals output from the B-pixels of the image pickup sensor 48.

The DSP 56 performs various types of signal processing, such as defect correction processing, offset processing, gain processing, color adjustment processing, gamma conversion processing, and demosaicing processing, on the received image signals. Signals of defective pixels of the image pickup sensor 48 are corrected in the defect correction processing. Dark current components are removed from the RGB image signals having been subjected to the defect correction processing in the offset processing, so that an accurate zero level is set.

The RGB image signals having been subjected to the offset processing are multiplied by a specific gain parameter in the gain processing, so that signal levels are adjusted. The specific gain parameter varies for each observation mode. For example, gain processing for normal light for multiplying the normal image signals by a gain parameter for normal light as the specific gain parameter is performed in the case of the mono-image display mode. Further, in the case of the analysis processing mode or the multi-image display mode, gain processing for first illumination light for multiplying the first image signals by a gain parameter for first illumination light as the specific gain parameter is performed, and gain processing for second illumination light for multiplying the second image signals by a gain parameter for second illumination light as the specific gain parameter is performed.

After that, brightness or a chroma saturation is adjusted by the gamma conversion processing. The demosaicing processing (also referred to as equalization processing or demosaicing) is performed on the RGB image signals having been subjected to linear matrix processing, so that signals of colors deficient in each pixel are generated by interpolation. All the pixels are made to have the signals of the respective colors of R, G, and B by this demosaicing processing.

The noise removing unit 58 performs noise removal processing (for example, a moving-average method, a median filtering method, or the like) on the RGB image signals, which have been subjected to gamma correction and the like by the DSP 56, to remove noise from the RGB image signals. The RGB image signals from which noise has been removed are transmitted to the image processing unit 60.

The image processing unit 60 performs various types of image processing on the RGB image signals. The various types of image processing vary for each mode. In a case where a mode is set to the mono-image display mode, a parameter to be used in the image processing unit 60 is switched to a color enhancement processing parameter for normal light and a structure enhancement processing parameter for normal light by the parameter switching unit 62. Then, the image processing unit 60 performs color enhancement processing for normal light on the normal image signals using the color enhancement processing parameter for normal light, and performs structure enhancement processing for normal light on the normal image signals using the structure enhancement processing parameter for normal light. After that, the normal image signals having been subjected to the above-mentioned processing are input to the display controller 66 as the normal image.

In a case where a mode is set to the analysis processing mode, a parameter to be used in the image processing unit 60 is switched to the color enhancement processing parameter for normal light and the structure enhancement processing parameter for normal light, which are used for the first image signals, and a parameter for analysis processing, which is used for the second image signals, by the parameter switching unit 62. The image processing unit 60 performs the color enhancement processing for normal light and the structure enhancement processing for normal light on the first image signals using the color enhancement processing parameter for normal light and the structure enhancement processing parameter for normal light. Accordingly, the normal image as a display image is obtained.

On the other hand, the image processing unit 60 performs analysis processing on the second image signals using the parameter for analysis processing. As the analysis processing, there is an artificial intelligence (AI) processing that outputs a specific output result in a case where a specific input image is output to a trained model subjected to machine learning. In addition, for example, the analysis processing includes blood vessel extraction processing for extracting shape information about blood vessels, index value calculation processing for calculating an index value for blood vessels on the basis of the extracted shape information about blood vessels, and the like. The result of the analysis processing is superimposed and displayed on the normal image, so that a display image with an analysis result is obtained. The display image with an analysis result is input to the display controller 66.

In a case where a mode is set to the multi-image display mode, parameters to be used in the image processing unit 60 are switched to a color enhancement processing parameter for first illumination light and a structure enhancement processing parameter for first illumination light, which are used for the first image signals, and a color enhancement processing parameter for second illumination light and a structure enhancement processing parameter for second illumination light, which are used for the second image signals, by the parameter switching unit 62. The image processing unit 60 performs color enhancement processing for first illumination light and structure enhancement processing for first illumination light on the first image signals using the color enhancement processing parameter for first illumination light and the structure enhancement processing parameter for first illumination light. Then, the first image signals having been subjected to the above-mentioned processing are input to the display controller 66 as the first observation image.

Further, the image processing unit 60 performs color enhancement processing for second illumination light and structure enhancement processing for second illumination light on the second image signals using the color enhancement processing parameter for second illumination light and the structure enhancement processing parameter for second illumination light. Furthermore, in a case where a mode is set to the multi-image display mode, the image processing unit 60 performs mucous membrane-color-balance processing for setting the colors of normal mucous membranes, which are included in the object to be observed, to the same color between the first observation image and the second observation image. First mucous membrane-color-balance processing is performed on the first observation image, and second mucous membrane-color-balance processing based on the result of the first mucous membrane-color-balance processing is performed on the second observation image. After that, the RGB image signals having been subjected to the above-mentioned processing are input to the display controller 66 as the second observation image.

B 1-image signals, G1-image signals, and R1-image signals included in the first observation image are automatically adjusted in the first mucous membrane-color-balance processing as described in, for example, D1) to D3) to be described below so that the average color of the entire screen has a specific color balance. The first mucous membrane-color-balance processing is performed on the assumption that the color of a mucous membrane is dominant over the object to be observed. Then, the first mucous membrane-color-balance processing is performed, so that B1^{∗}-image signals, G1^{∗}-image signals, and R1^{∗}-image signals having been subjected to the first mucous membrane-color-balance processing are obtained.
D1) B1^{∗}-image signal=B1/B1ave
D2) G1^{∗}-image signal=G1/G1ave
D3) R1^{∗}-image signal=R1/R1ave

Here, B1ave denotes the average pixel value of the B 1-image signals (the sum of pixel values of the entire screen (effective pixels)/the number of effective pixels). Glave denotes the average pixel value of the G1-image signals (the sum of pixel values of the entire screen (effective pixels)/the number of effective pixels). Rlave denotes the average pixel value of the R1-image signals (the sum of pixel values of the entire screen (effective pixels)/the number of effective pixels).

Further, B2-image signals, G2-image signals, and R2-image signals included in the second observation image are automatically adjusted in the second mucous membrane-color-balance processing as described in, for example, E1) to E3) to be described below so that the average color of the entire screen has a specific color balance. B1ave, G1ave, and Rlave calculated in the first mucous membrane-color-balance processing are used in the second mucous membrane-color-balance processing. Then, the second mucous membrane-color-balance processing is performed, so that B2^{∗}-image signals, G2^{∗}-image signals, and R2^{∗}-image signals having been subjected to the second mucous membrane-color-balance processing are obtained.
E1) B2^{∗}-image signal=B2/B1ave
E2) G2^{∗}-image signal=G2/G1ave
E3) R2^{∗}-image signal=R2/R1ave

The display controller 66 performs a control to display the normal image, the display image with an analysis result, the first observation image, or the second observation image, which is input from the image processing unit 60, as an image that can be displayed on the display 18. In the case of the mono-image display mode, the display controller 66 causes the display 18 to display the normal image.

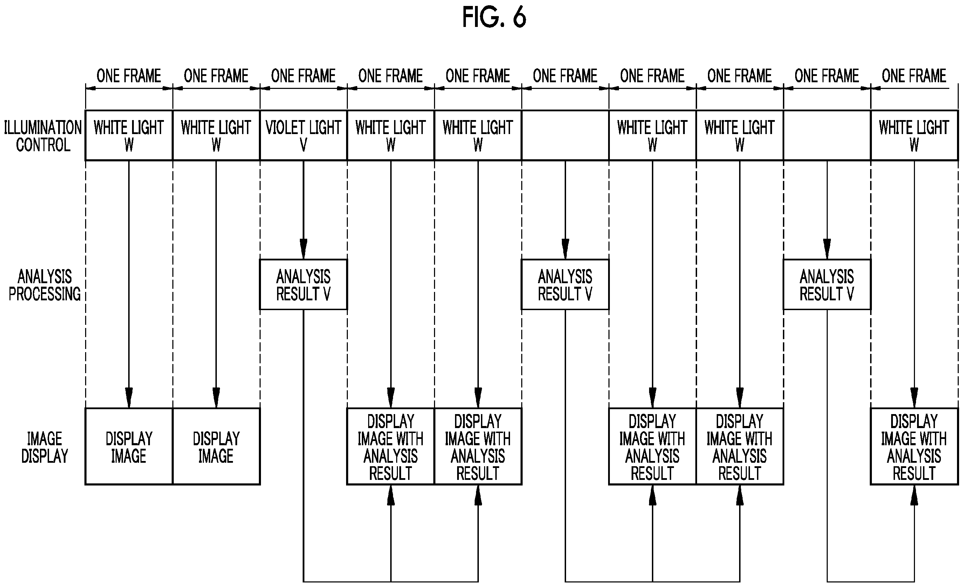

In a case where the first light emission pattern is set to the first A light emission pattern and the second light emission pattern is set to the second A pattern (the number of frames of the second illumination period is the same and the emission spectrum of the second illumination light is the same) in the case of the analysis processing mode and the object to be observed is illuminated with white light W as the first illumination light for two frames by only one frame between the emission of violet light V as the second illumination light and the emission of violet light V, analysis processing is performed on the R2-image signals, the G2-image signals, and the B2-image signal that are obtained from illumination using violet light V, so that an analysis result V is obtained as shown in Fig. 6. The analysis result V is superimposed and displayed on the display image, and the display image is displayed on the display 18 as the display image with an analysis result.

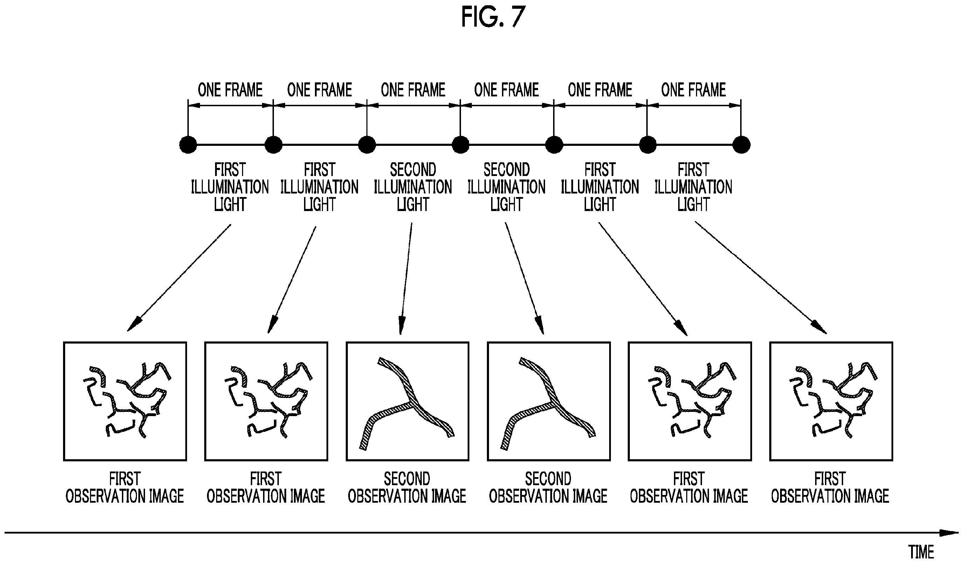

In a case where the first light emission pattern is set to the first A light emission pattern and the second light emission pattern is set to the second A pattern (the number of frames of the second illumination period is the same and the emission spectrum of the second illumination light is the same) in the case of the multi-image display mode and the first illumination light is emitted for two frames and the second illumination light is emitted for two frames, the display controller 66 causes the display 18 to display the first observation image or the second observation image while being switched at an interval of two frames as shown in Fig. 7.

The central controller 68 controls the respective parts of the processor device 16 in addition to executing the program as described above. Further, the central controller 68 receives information from the endoscope 12 or the light source device 14, and performs the control of the respective parts of the processor device 16 and the control of the endoscope 12 or the light source device 14 on the basis of the received information.

The automatic mode switching unit 69 automatically switches mono-light emission and multi-light emission on the basis of the activation or non-activation of the analysis processing mode, the activation or non-activation of the multi-image display mode, and a predetermined light source switching condition. A switching control performed by the automatic mode switching unit 69 is divided into three patterns of a case where the analysis processing mode is not activated (OFF) and the multi-image display mode is activated (ON) (switching pattern A), a case where the analysis processing mode is activated (ON) and the multi-image display mode is not activated (OFF) (switching pattern B), and a case where the analysis processing mode is activated (ON) and the multi-image display mode is activated (ON) (switching pattern C).

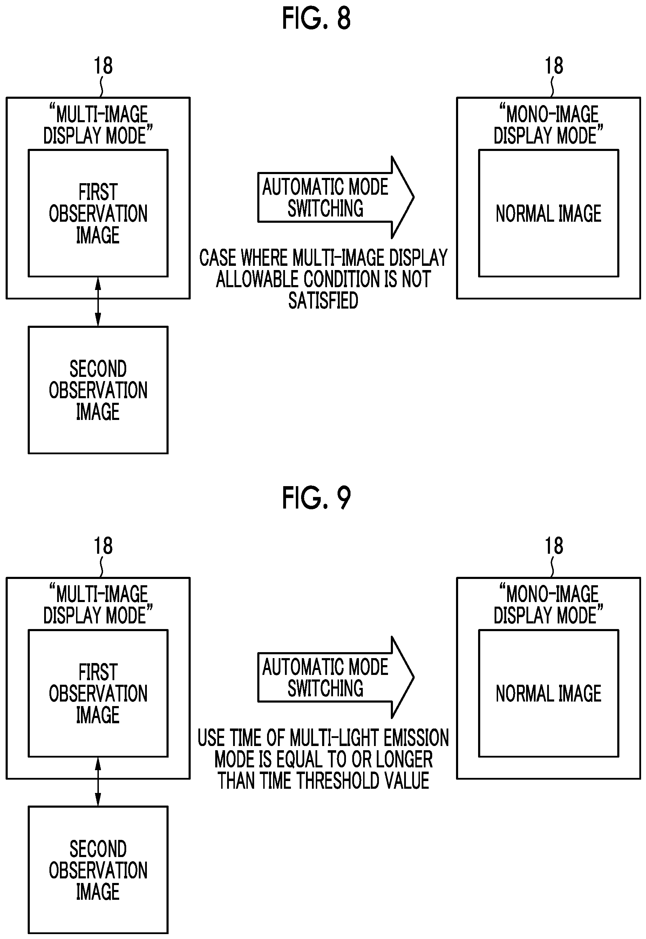

In the case of the switching pattern A (analysis processing mode: OFF, multi-image display mode: ON), the automatic mode switching unit 69 automatically switches light emission to the multi-light emission from the mono-light emission during the activation of the mono-light emission as the multi-image display mode is switched to ON from OFF. In a case where a preset multi-image display allowable condition (light source switching condition) is satisfied, the automatic mode switching unit 69 continues the multi-light emission in the multi-image display mode where the multi-light emission is performed. On the other hand, in a case where the multi-image display allowable condition is not satisfied, the automatic mode switching unit 69 automatically switches light emission to the mono-light emission from the multi-light emission. In this case, as shown in Fig. 8, the automatic mode switching unit 69 performs processing for switching a mode to a state where only the normal image is continuously displayed on the display 18 from a state where the first observation image and the second observation image are displayed on the display 18 while being automatically switched. That is, a mode is automatically switched to the mono-image display mode.

Since there is a case where it is difficult for a user to know to which one of the multi-image display mode and the mono-image display mode a mode is set, the display 18 displays that a mode is set to the "multi-image display mode" in the case of the multi-light emission and displays that a mode is set to the "mono-image display mode" in the case of the mono-light emission.

A case where the multi-image display allowable condition is not satisfied is that, for example, the use time of the multi-image display mode is equal to or longer than a predetermined time threshold value. In this case, a time, which has passed after a mode is set to the multi-image display mode by the mode changeover SW 13a, is measured by a time measurement unit (not shown) provided in the processor device 16. Then, in a case where the measured time is equal to or longer than the time threshold value, light emission is automatically switched to the mono-light emission from the multi-light emission as shown in Fig. 9. Accordingly, a mode is automatically switched to the mono-image display mode from the multi-image display mode.

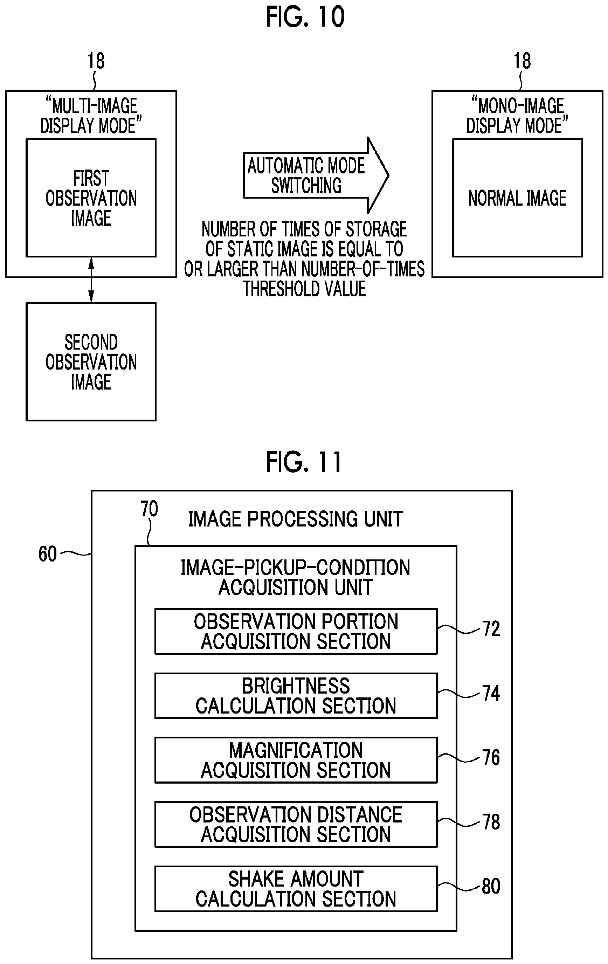

Further, a case where the multi-image display allowable condition is not satisfied is that, for example, the number of times of storage of the static image of the object to be observed is equal to or larger than a predetermined number-of-times threshold value. In this case, the number of times of operation of the static-image-acquisition instruction part 13b is counted by a number-of-times counter unit (not shown) provided in the processor device 16. Then, in a case where the counted number of times is equal to or larger than the number-of-times threshold value, light emission is automatically switched to the mono-light emission from the multi-light emission as shown in Fig. 10. Accordingly, a mode is automatically switched to the mono-image display mode from the multi-image display mode.

Furthermore, it is preferable that a case where the multi-image display allowable condition is not satisfied is a case where image pickup conditions about the object to be observed are changed. In this case, an image-pickup-condition acquisition unit 70 is provided in the image processing unit 60 of the processor device 16 to acquire the image pickup conditions. As shown in Fig. 11, the image-pickup-condition acquisition unit 70 comprises an observation portion acquisition section 72, a brightness calculation section 74, a magnification acquisition section 76, an observation distance acquisition section 78, and a shake amount calculation section 80.

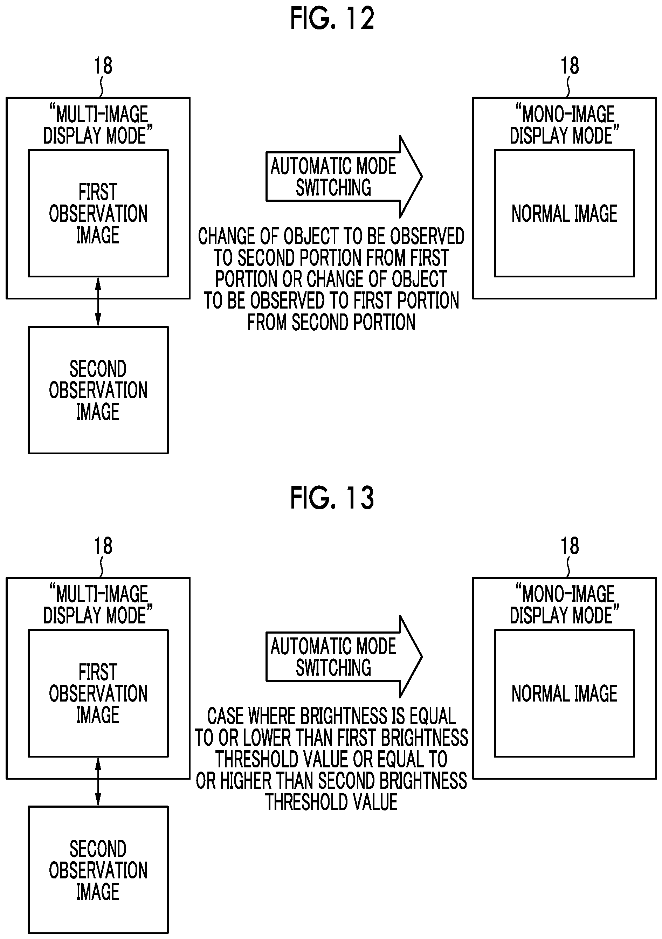

In a case where a case where the multi-image display allowable condition is not satisfied is that an observation portion, which is one of the image pickup conditions, is changed, for example, an observation portion of which the image is picked up at present is changed to a second portion (for example, "stomach") from a first portion (for example, "gullet") or the observation portion is changed to the first portion from the second portion, light emission is automatically switched to the mono-light emission from the multi-light emission as shown in Fig. 12. Since it is thought that the distal end part 12d of the endoscope is moved in a case where the observation portion is changed, the object to be observed may not be reliably illuminated with the first illumination light and the second illumination light that are alternately emitted. For this reason, such a case is not suitable for the multi-light emission.

Information about the observation portion is acquired by the observation portion acquisition section 72. The observation portion acquisition section 72 determines the observation portion from the image feature quantity of the first observation image or the second observation image obtained in the multi-light emission. For example, in a case where the brightness of the central portion of the screen is lower than that of a peripheral portion thereof in the first observation image or the second observation image, the observation portion is determined as the "gullet". Further, in a case where the brightness of the central portion of the screen is higher than that of a peripheral portion thereof in the first observation image or the second observation image, the observation portion is determined as the "stomach".

In a case where a case where the multi-image display allowable condition is not satisfied is that the brightness of the object to be observed, which is one of the image pickup conditions, is equal to or lower than a first brightness threshold value or is equal to or higher than a second brightness threshold value larger than the first brightness threshold value, light emission is automatically switched to the mono-light emission from the multi-light emission as shown in Fig. 13. Accordingly, a mode is automatically switched to the mono-image display mode from the multi-image display mode. In a case where the brightness is equal to or lower than the first brightness threshold value, the entire object to be observed is dark. For this reason, such a case is not suitable for the multi-light emission. Likewise, in a case where the brightness is equal to or higher than the second brightness threshold value, the entire object to be observed is extremely bright as in a case where halation occurs, and the like. For this reason, such a case is not suitable for the multi-light emission. Information about the brightness is acquired by the brightness calculation section 74. The brightness calculation section 74 calculates the average value of pixel values from the first observation image or the second observation image, and calculates brightness from the calculated average value of pixel values. Here, as the average value of pixel values is larger, brightness is higher.

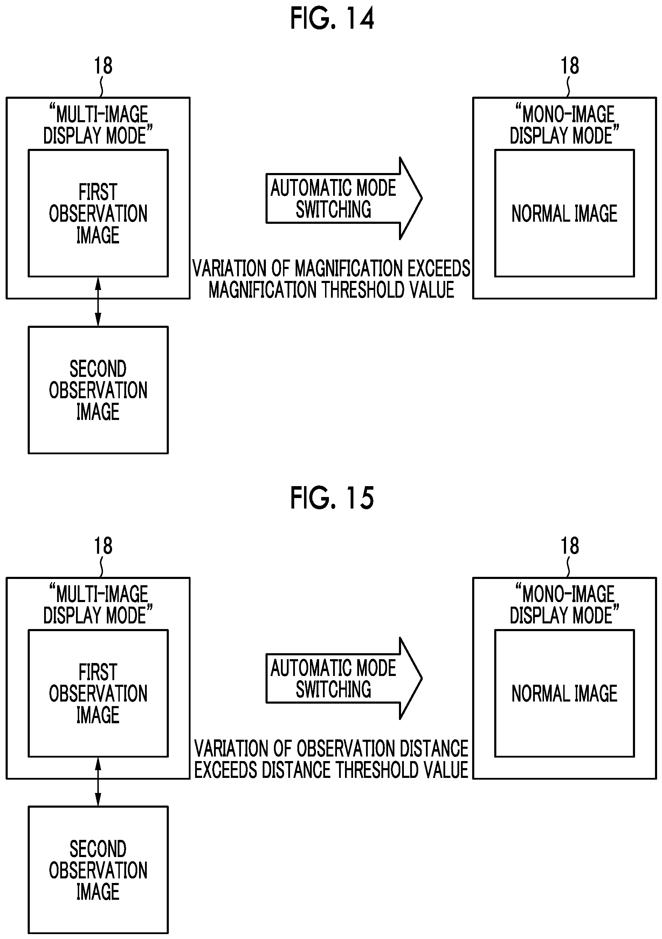

In a case where a case where the multi-image display allowable condition is not satisfied is that the variation of the magnification of the object to be observed, which is one of the image pickup conditions, exceeds a magnification threshold value, light emission is automatically switched to the mono-light emission from the multi-light emission as shown in Fig. 14. Accordingly, a mode is automatically switched to the mono-image display mode from the multi-image display mode. In a case where the magnification of the object to be observed is significantly changed so that the variation of magnification exceeds the magnification threshold value, the illumination distribution of illumination light on the object to be observed is changed. For this reason, such a case is often not suitable for the multi-light emission. With regard to the magnification of the object to be observed, zoom information representing which the magnification of the object to be observed is set to is transmitted to the magnification acquisition section 76 whenever the zoom operation part 13c is operated. The automatic mode switching unit 69 determines whether or not the variation of magnification exceeds the magnification threshold value with reference to the zoom information acquired by the magnification acquisition section 76.

In a case where a case where the multi-image display allowable condition is not satisfied is that the variation of an observation distance (a distance between the distal end part 12d of the endoscope and the object to be observed), which is one of the image pickup conditions, exceeds a distance threshold value, light emission is automatically switched to the mono-light emission from the multi-light emission as shown in Fig. 15. Accordingly, a mode is automatically switched to the mono-image display mode from the multi-image display mode. As with the magnification of the object to be observed, in a case where the observation distance is significantly changed so that the variation of the observation distance exceeds the distance threshold value, the illumination distribution of illumination light on the object to be observed is changed. For this reason, such a case is often not suitable for the multi-light emission. With regard to the observation distance, the observation distance acquisition section 78 calculates the average value of pixel values from the first observation image or the second observation image obtained in the multi-light emission and obtains an observation distance from the calculated average value of pixel values. Here, as the average value of pixel values is larger, the observation distance is shorter.

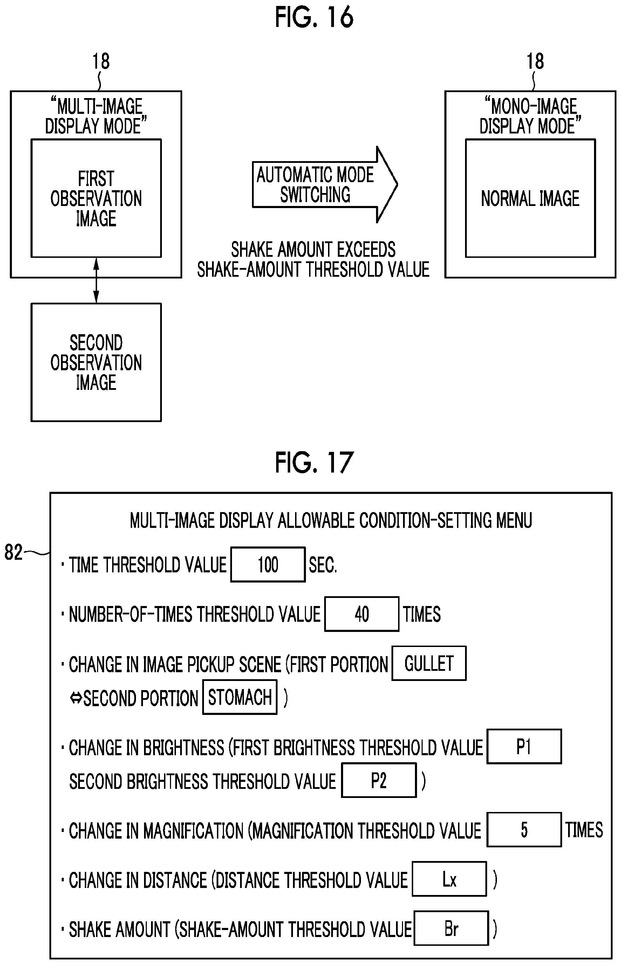

In a case where a case where the multi-image display allowable condition is not satisfied is that the shake amount of the image, which is one of the image pickup conditions, exceeds a shake-amount threshold value, light emission is automatically switched to the mono-light emission from the multi-light emission as shown in Fig. 16. Accordingly, a mode is automatically switched to the mono-image display mode from the multi-image display mode. In a case where the shake amount of the image is large and exceeds the shake-amount threshold value, illumination light may not reliably reach the object to be observed. For this reason, such a case is often not suitable for the multi-light emission. With regard to the shake amount, the shake amount calculation section 80 obtains contrast from the first observation image or the second observation image obtained in the multi-light emission and calculates a shake amount from the contrast. As the contrast is lower, the shake amount is larger. A method of calculating the shake amount from the frequency component of the first observation image or the second observation image may be used as a method of calculating the shake amount in addition to a method using contrast (the shake amount is increased as the frequency component is a lower frequency component).

The multi-image display allowable condition that is used to automatically switch light emission to the mono-light emission from the multi-light emission can be appropriately set by a user as described above. In this case, a user operates the user interface 19 to cause the display 18 to display a multi-image display allowable condition-setting menu 82 shown in Fig. 17. The "time threshold value" and the "number-of-times threshold value" can be set in the multi-image display allowable condition-setting menu 82. In a case where the "time threshold value" is set to "100 sec." in the multi-image display allowable condition-setting menu 82, light emission is automatically switched to the mono-light emission from the multi-light emission at a point of time when the use time of the multi-image display mode reaches "100 sec." Further, in a case where the "number-of-times threshold value" is set to "40 times" in the multi-image display allowable condition-setting menu 82, light emission is automatically switched to the mono-light emission from the multi-light emission at a point of time when the number of times of operation of the static-image-acquisition instruction part 13b reaches "40 times".

It is preferable that the same multi-image display restart condition-setting menu as the multi-image display allowable condition-setting menu 82 is displayed on the display 18 so that a multi-image display restart condition used for the automatic switching of the mono-light emission to the multi-light emission can be appropriately set as described later.

Further, the "first portion" and the "second portion" can be set in the multi-image display allowable condition-setting menu 82. In a case where the "first portion" is set to the "gullet" and the "second portion" is set to the "stomach" and the observation portion is changed to the "stomach" from the "gullet" or the observation portion is changed to the "gullet" from the "stomach", light emission is automatically switched to the mono-light emission from the multi-light emission. Furthermore, the "first brightness threshold value" and the "second brightness threshold value" can be set in the multi-image display allowable condition-setting menu 82. In a case where the "first brightness threshold value" is set to P1 and the "second brightness threshold value" is set to P2 (>P1) and the brightness of the object to be observed is equal to or lower than P1 or is equal to or higher than P2, light emission is automatically switched to the mono-light emission to the multi-light emission.

Further, the "magnification threshold value" can be set in the multi-image display allowable condition-setting menu 82. In a case where the "magnification threshold value" is set to "5 times", light emission is automatically switched to the mono-light emission from the multi-light emission at a point of time when the variation of magnification of the object to be observed exceeds "5 times". Furthermore, the "distance threshold value" can be set in the multi-image display allowable condition-setting menu 82. In a case where the "distance threshold value" is set to Lx and the variation of the observation distance exceeds Lx, light emission is automatically switched to the mono-light emission from the multi-light emission. Moreover, the "shake-amount threshold value" can be set in the multi-image display allowable condition-setting menu 82. In a case where the "shake-amount threshold value" is set to Br and the shake amount exceeds Br, light emission is automatically switched to the mono-light emission from the multi-light emission.

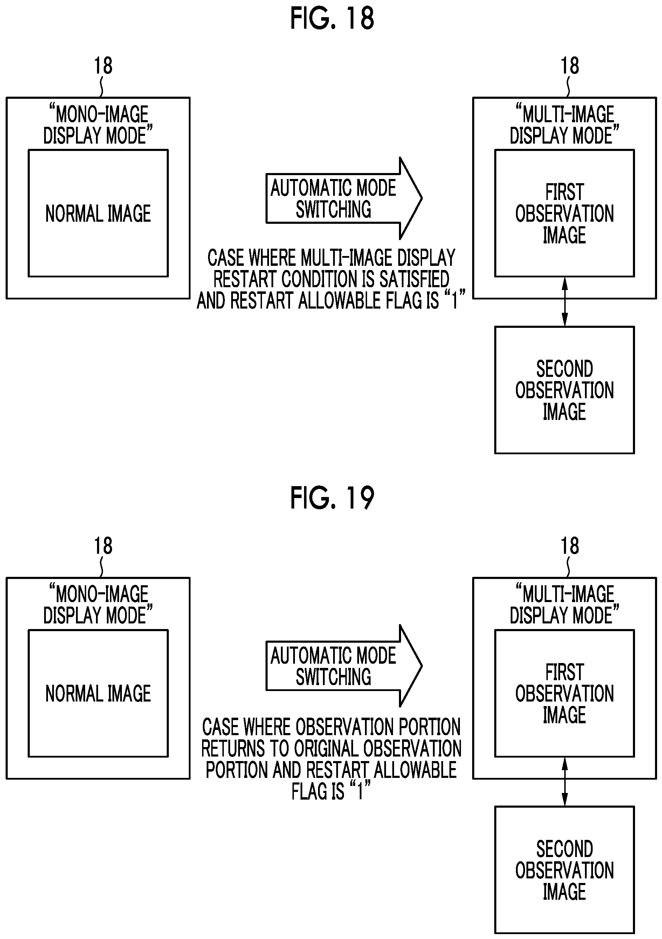

In a case where light emission is automatically switched to the mono-light emission from the multi-light emission and a mode is automatically switched to the mono-image display mode from the multi-image display mode since the multi-image display allowable condition is not satisfied, the automatic mode switching unit 69 automatically switches light emission to the multi-light emission from the mono-light emission and activates the multi-image display mode as shown in Fig. 18 in a case where a preset multi-image display restart condition (light source switching condition) is satisfied. The automatic mode switching unit 69 automatically switches light emission to the mono-light emission in a case where the image pickup condition about the object to be observed is changed, and the like. However, it is preferable that the automatic mode switching unit 69 automatically switches light emission to the multi-light emission from the mono-light emission in a case where a change in the image pickup condition about the object to be observed is within a multi-light emission allowable range, and the like, that is, in a case where the multi-image display restart condition is satisfied, and the like. "A change in the image pickup condition is within a multi-light emission allowable range" means that the visibility of the switching display of the first observation image and the second observation image obtained in the multi-light emission is not affected in spite of a change in the image pickup condition.

It is preferable that the multi-image display restart condition is a case where the object to be observed returns to the first portion within a certain period of time after being changed to the second portion (for example, "stomach ") from the first portion (for example, "gullet") or a case where the object to be observed returns to the second portion within a certain period of time after being changed to the first portion from the second portion. In this case, light emission is automatically switched to the multi-light emission from the mono-light emission as shown in Fig. 19. Accordingly, a mode is automatically switched to the multi-image display mode from the mono-image display mode. The reason for this is that an illumination condition is not changed so much and a light emission state caused by the multi-light emission is not affected in a case where the observation portion immediately returns to the original portion. Information about the observation portion is acquired by the observation portion acquisition section 72 as described above.

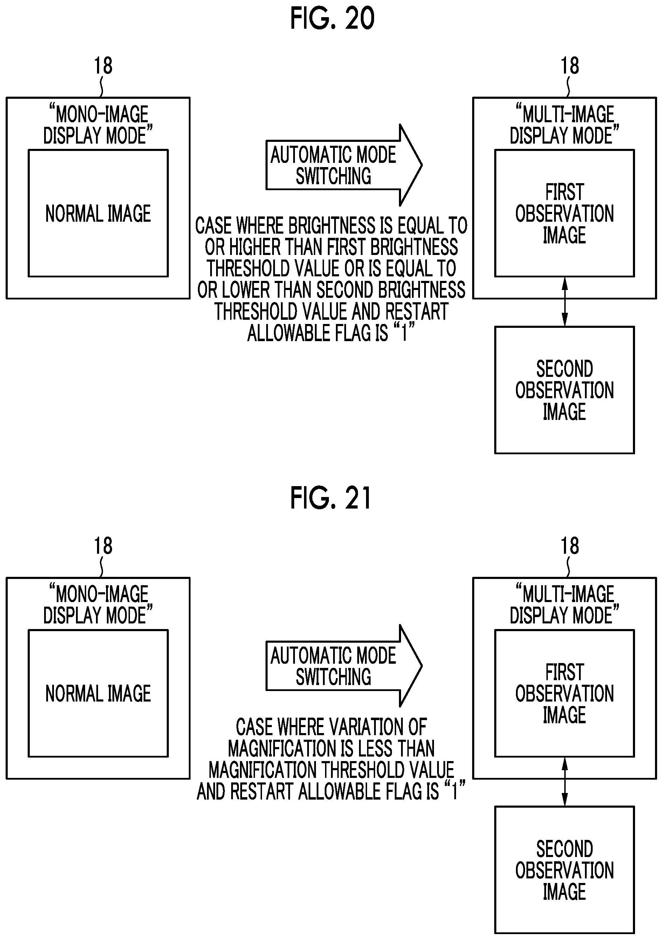

It is preferable that the multi-image display restart condition is a case where the brightness of the object to be observed is equal to or higher than the first brightness threshold value or is equal to or lower than the first brightness threshold value. In this case, light emission is automatically switched to the multi-light emission from the mono-light emission as shown in Fig. 20. Accordingly, a mode is automatically switched to the multi-image display mode from the mono-image display mode. The reason for this is that a light emission state caused by the multi-light emission is not affected in a case where the brightness returns to original normal brightness (equal to or higher than the first brightness threshold value or equal to or lower than the first brightness threshold value) after a certain period of time even though the brightness is reduced or increased. Information about the brightness is acquired by the brightness calculation section 74 as described above.

It is preferable that the multi-image display restart condition is that the variation of magnification of the object to be observed is less than the magnification threshold value in a case where the magnification of the object to be observed is changed. In this case, light emission is automatically switched to the multi-light emission from the mono-light emission as shown in Fig. 21. Accordingly, a mode is automatically switched to the multi-image display mode from the mono-image display mode. The reason for this is that a light emission state caused by the multi-light emission is not affected in a case where a change in the magnification of the object to be observed is not so large and the variation of magnification temporarily exceeds the magnification threshold value and immediately falls below the magnification threshold value. The magnification of the object to be observed is acquired by the magnification acquisition section 76 as described above.

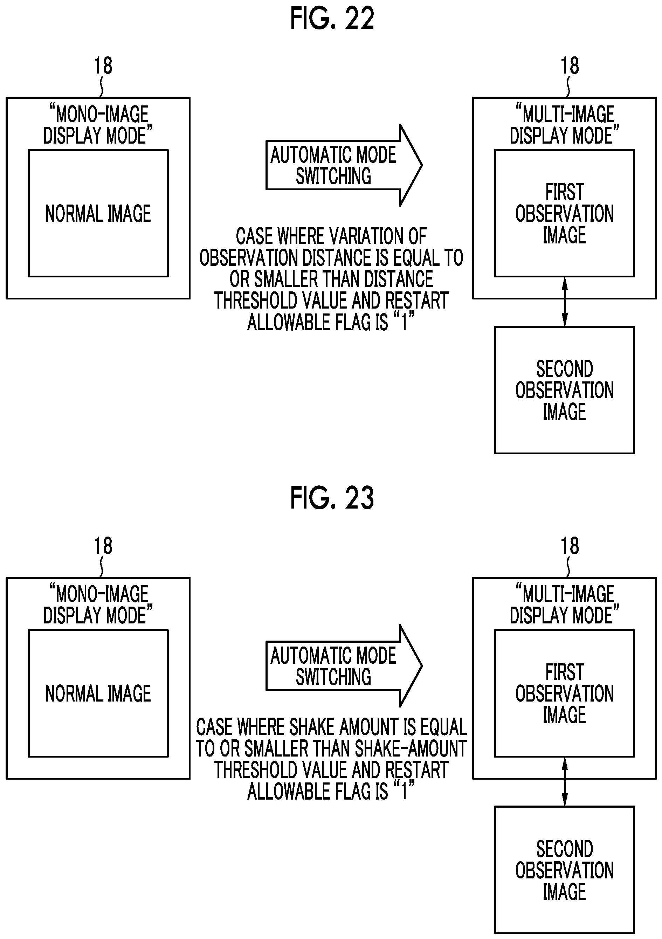

It is preferable that the multi-image display restart condition is a case where the variation of an observation distance is equal to or smaller than the distance threshold value. In this case, light emission is automatically switched to the multi-light emission from the mono-light emission as shown in Fig. 22. Accordingly, a mode is automatically switched to the multi-image display mode from the mono-image display mode. The reason for this is that a light emission state caused by the multi-light emission is not affected in a case where a change in an observation distance is not so large and the variation of an observation distance temporarily exceeds the distance threshold value and immediately falls below the distance threshold value. An observation distance is acquired by the observation distance acquisition section 78 as described above.

It is preferable that the multi-image display restart condition is a case where the shake amount of an observation image is equal to or smaller than the shake-amount threshold value. In this case, light emission is automatically switched to the multi-light emission from the mono-light emission as shown in Fig. 23. Accordingly, a mode is automatically switched to the multi-image display mode from the mono-image display mode. The reason for this is that a light emission state caused by the multi-light emission is not affected in a case where the shake amount of an image is not so large and the shake amount temporarily exceeds the shake-amount threshold value and immediately falls below the shake-amount threshold value. The shake amount is acquired by the shake amount calculation section 80 as described above.

However, in order to prevent light emission from being automatically switched to the multi-light emission (in order to prevent a mode from being automatically switched to the multi-image display mode) unintentionally, the automatic mode switching unit 69 automatically switches light emission to the multi-light emission from the mono-light emission and automatically switches a mode to the multi-image display mode from the mono-image display mode in a case where the multi-image display restart condition is satisfied and a restart allowable condition is satisfied. On the other hand, it is preferable that the automatic mode switching unit 69 prohibits light emission from being automatically switched to the multi-light emission in a case where the multi-image display restart condition is satisfied and the restart allowable condition (light source switching condition) is not satisfied. It is preferable that the restart allowable condition can be set by a user. Further, a restart allowable/unallowable flag representing the restart allowable condition is used in order to determine whether or not the restart allowable condition is satisfied. In a case where a restart allowable/unallowable flag is "1", it means that the restart allowable condition is not satisfied. In a case where a restart allowable/unallowable flag is "0", it means that the restart allowable condition is satisfied (see Figs. 18 to 23).

It is preferable that the restart allowable condition is a case where the analysis processing mode is OFF and a user does not select the mono-image display mode using the mode changeover SW 13a. In a case where a user selects the mono-light emission, a restart allowable/unallowable flag is "0". In a case where a user does not select the mono-light emission, a restart allowable/unallowable flag is "1". It is preferable that light emission is not automatically switched to the multi-light emission in a case where a user voluntarily selects the mono-image display mode.

It is preferable that the restart allowable condition is a case where the analysis processing mode is OFF and a restart allowable time does not elapse yet after the multi-image display allowable condition is satisfied and light emission is automatically switched to the mono-light emission from the multi-light emission. In this case, a time elapsed after light emission is switched to the mono-light emission is measured by the time measurement unit (not shown). In a case where the measured time is shorter than the restart allowable time, the restart allowable/unallowable flag is maintained at "1". Further, in a case where the measured time is equal to or longer than the restart allowable time, the restart allowable/unallowable flag is switched to "0". Since it is thought that a state after the restart allowable time has elapsed is suitable for an observation based on the mono-light emission, it is preferable that light emission is not automatically switched to the multi-light emission.

It is preferable that the restart allowable condition is a case where the analysis processing mode is OFF and the multi-light emission is implementable. Whether the multi-light emission can be implemented or not is determined by a multi-light emission-enablement-determination unit (not shown) of the processor device 16. Specifically, the multi-light emission-enablement-determination unit detects abnormalities and the like related to the endoscope 12, the light source device 14, and the processor device 16, and determines whether the multi-light emission can be implemented or not on the basis of detection results. In this case, the restart allowable/unallowable flag is set to "1" in a case where the multi-light emission-enablement-determination unit determines that the multi-light emission can be implemented, and the restart allowable/unallowable flag is set to "0" in a case where the multi-light emission-enablement-determination unit determines that the multi-light emission cannot be implemented. In a case where abnormalities and the like related to the endoscope 12, the light source device 14, and the processor device 16 occur and it is difficult to implement the multi-light emission, it is preferable that light emission is not automatically switched to the multi-light emission.

It is preferable that the restart allowable condition is a case where the analysis processing mode is OFF and the use or non-use of the magnification change unit is not switched. The use of the magnification change unit means a state where the zoom operation part 13c is set to "ON" so that a magnification can be changed. The non-use of the magnification change unit means a state where the zoom operation part 13c is set to "OFF" so that a magnification is not changed. In this case, after the multi-image display allowable condition is satisfied and light emission is automatically switched to the mono-light emission, the restart allowable/unallowable flag is set to "1" in a case where the use or non-use of the magnification change unit is not switched but the restart allowable/unallowable flag is set to "0" in a case where the use or non-use of the magnification change unit is switched. In a case where the use or non-use of the magnification change unit is switched, an object different from that in the case of the previous multi-light emission is observed and it is preferable that light emission is not automatically switched to the multi-light emission since the multi-light emission is not necessarily used.

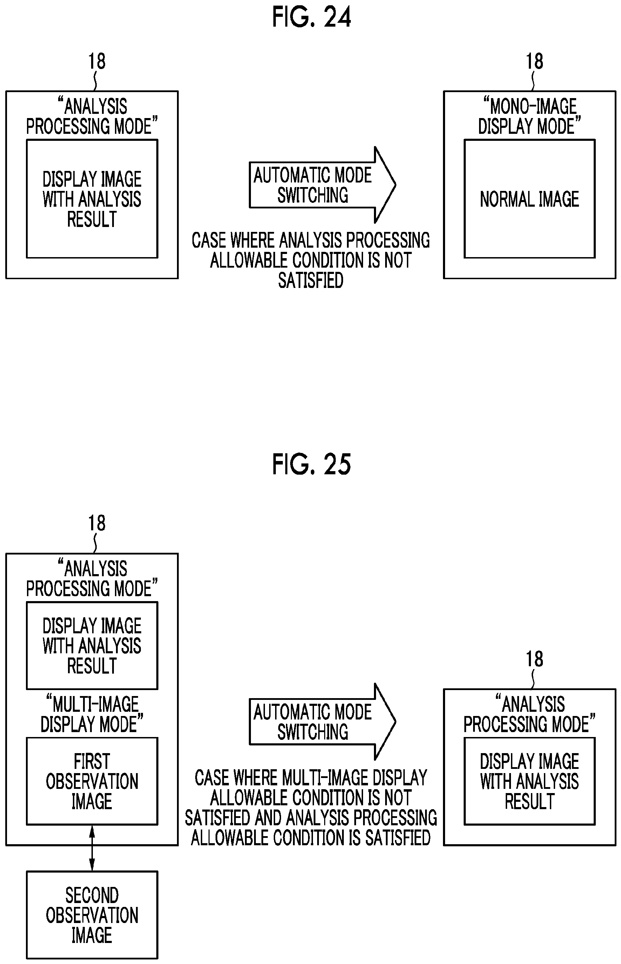

In the case of the switching pattern B (analysis processing mode: ON, multi-image display mode: OFF), the automatic mode switching unit 69 automatically switches light emission to the multi-light emission from the mono-light emission during the activation of the mono-light emission as the analysis processing mode is switched to ON from OFF. In a case where a preset analysis processing allowable condition (light source switching condition) is satisfied, the automatic mode switching unit 69 prohibits light emission from being automatically switched to the mono-light emission from the multi-light emission in the analysis processing mode where the multi-light emission is performed. On the other hand, in a case where the analysis processing allowable condition is not satisfied, the automatic mode switching unit 69 automatically switches light emission to the mono-light emission from the multi-light emission. Accordingly, as shown in Fig. 24, a mode is automatically switched to the mono-image display mode where only the normal image is displayed on the display 18 from the analysis processing mode where the display image with an analysis result is displayed on the display 18.

It is preferable that the analysis processing allowable condition is that, for example, a change in the image pickup condition about the object to be observed is within an analysis processing allowable range. "Within an analysis processing allowable range" means that analysis processing can be performed with certain accuracy in spite of a change in the image pickup condition. A specific example of "within an analysis processing allowable range" is a case where an area occupied by a region, which can affect analysis processing, such as halation or a dark portion, in a screen based on the second image signals is equal to or smaller than a certain area. On the other hand, a specific example of "out of an analysis processing allowable range" is a case where an area occupied by a region, which can affect analysis processing, such as halation or a dark portion, in the screen based on the second image signals exceeds than a certain area.

In a case where a mode is automatically switched to the mono-image display mode from the analysis processing mode and a predetermined analysis processing restart condition (light source switching condition) is satisfied, the automatic mode switching unit 69 may automatically switch a mode to the analysis processing mode from the mono-image display mode. In this case, it is preferable that the automatic mode switching unit 69 prohibits a mode from being automatically switched to the analysis processing mode in a case where the restart allowable condition (which is preferably the same as described above) is not satisfied even though the analysis processing restart condition is satisfied. The analysis processing restart condition may be the same as or different from the multi-image display restart condition.

In the case of the switching pattern C (analysis processing mode: ON, multi-image display mode: ON), the automatic mode switching unit 69 automatically switches light emission to the multi-light emission from the mono-light emission during the activation of the mono-light emission as the analysis processing mode and the multi-image display mode are switched to ON from OFF. Accordingly, the display image with an analysis result is displayed on the display 18 and the first observation image and the second observation image are displayed on the display 18 while being automatically switched (see Figs. 25 to 27).

In a case where the analysis processing allowable condition is satisfied even though the multi-image display allowable condition is not satisfied, the automatic mode switching unit 69 prohibits light emission from being automatically switched to the mono-light emission from the multi-light emission in the analysis processing mode and the multi-image display mode where the multi-light emission is performed. In this case, as shown in Fig. 25, the automatic switching display of the first observation image and the second observation image is stopped on the display 18 but the display of the display image with an analysis result on the display 18 is continued.

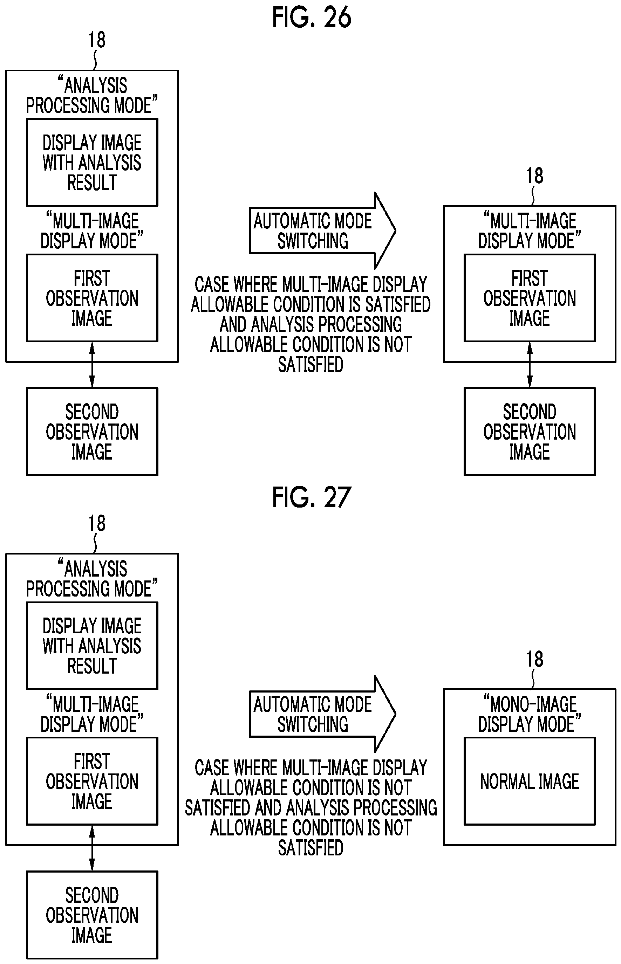

Further, in a case where the analysis processing allowable condition is not satisfied even though the multi-image display allowable condition is satisfied, the automatic mode switching unit 69 prohibits light emission from being automatically switched to the mono-light emission from the multi-light emission in the analysis processing mode and the multi-image display mode where the multi-light emission is performed. In this case, as shown in Fig. 26, the display of the display image with an analysis result on the display 18 is stopped but the automatic switching display of the first observation image and the second observation image on the display 18 is continued.

Furthermore, in a case where the multi-image display allowable condition is not satisfied and the analysis processing allowable condition is not satisfied, the automatic mode switching unit 69 automatically switches light emission to the mono-light emission from the multi-light emission in the analysis processing mode and the multi-image display mode where the multi-light emission is performed. Accordingly, as shown in Fig. 27, a mode is switched to the mono-image display mode where only the normal image is displayed on the display 18.

Even in the switching pattern C, the automatic mode switching unit 69 may automatically switch a mode to the analysis processing mode from the mono-image display mode in a case where a mode is automatically switched to the mono-image display mode from the analysis processing mode and the predetermined analysis processing restart condition is satisfied. It is preferable that the automatic mode switching unit 69 prohibits a mode from being automatically switched to the analysis processing mode in a case where the restart allowable condition is not satisfied even though the analysis processing restart condition is satisfied.

Further, even in the switching pattern C, the automatic mode switching unit 69 may automatically switch a mode to the multi-image display mode from the mono-image display mode in a case where a mode is automatically switched to the mono-image display mode from the multi-image display mode and the predetermined multi-image display restart condition is satisfied. It is preferable that the automatic mode switching unit 69 prohibits a mode from being automatically switched to the multi-image display mode in a case where the restart allowable condition is not satisfied even though the multi-image display restart condition is satisfied.

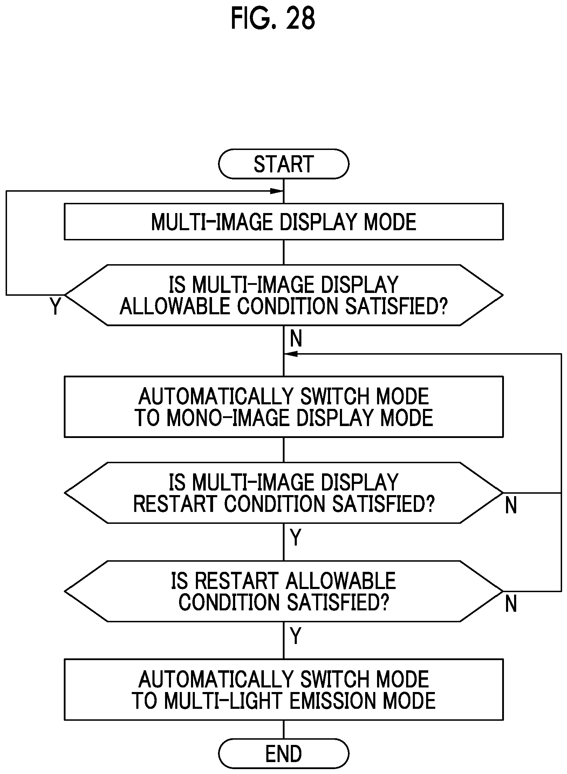

Next, a series of flows in the case of the switching pattern A (analysis processing mode: OFF, multi-image display mode: ON) will be described with reference to a flowchart shown in Fig. 28. In the multi-image display mode, the first illumination light and the second illumination light are emitted while being switched according to a specific light emission pattern (in this embodiment, at an interval of two frames). Further, the first observation image obtained from the image pickup of an object to be observed illuminated with the first illumination light and the second observation image obtained from the image pickup of the object to be observed illuminated with the second illumination light are displayed on the display 18 while being switched according to a specific display pattern (in this embodiment, at an interval of two frames).

As long as the multi-image display allowable condition is satisfied, the multi-image display mode is continued. Further, in a case where the predetermined multi-image display allowable condition is not satisfied, light emission is automatically switched to the mono-light emission from the multi-light emission. Accordingly, a mode is automatically switched to the mono-image display mode from the multi-image display mode.

A case where the multi-image display allowable condition is not satisfied includes a case where the use time of the multi-light emission exceeds the time threshold value, a case where the number of times of storage of a static image exceeds the number-of-times threshold value, and the like. Moreover, a case where the multi-image display allowable condition is not satisfied includes a case where the image pickup conditions about the object to be observed are changed. In a case where light emission is automatically switched to the mono-light emission (the switching of a mode to the mono-image display mode), the switching and emission of the first illumination light and the second illumination light are stopped. According to this, the switching display of the first and second observation images is also stopped. Then, the normal light is emitted and the normal image obtained from the image pickup of the object to be observed illuminated with the normal light is displayed on the display 18.

After a mode is automatically switched to the mono-image display mode, the processor device 16 monitors whether or not the multi-image display restart condition is satisfied. In a case where the multi-image display restart condition is satisfied and the restart allowable condition is satisfied, light emission is automatically switched to the multi-light emission from the mono-light emission. Accordingly, a mode is switched to the multi-image display mode from the mono-image display mode. On the other hand, in a case where the restart allowable condition is not satisfied even though the multi-image display restart condition is satisfied, the automatic switching of light emission to the multi-light emission is prohibited. Further, even in a case where the multi-image display restart condition is not satisfied, the automatic switching of light emission to the multi-light emission is prohibited. In a case where the automatic switching of light emission to the multi-light emission is prohibited, the mono-image display mode is continued.

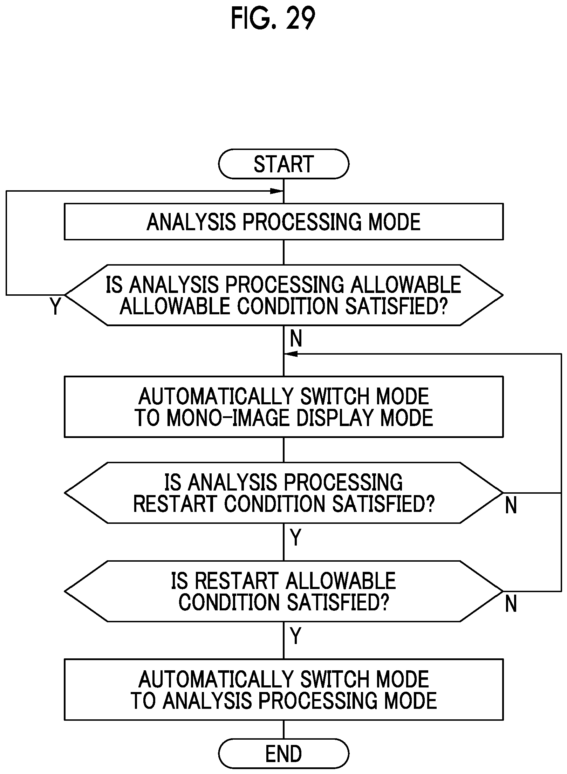

Next, a series of flows in the case of the switching pattern B (analysis processing mode: ON, multi-image display mode: OFF) will be described with reference to a flowchart shown in Fig. 29. In the case of the analysis processing mode, the first illumination light and the second illumination light are emitted while being switched according to a specific light emission pattern. Further, analysis processing is performed on the second image signals that are obtained from the image pickup of an object to be observed illuminated with the second illumination light. Furthermore, the analysis result of the analysis processing is superimposed and displayed on a display image based on the first image signals obtained from the image pickup of the object to be observed illuminated with the first illumination light, so that a display image with an analysis result is obtained. The display image with an analysis result is displayed on the display 18.

As long as the analysis processing allowable condition is satisfied, the analysis processing mode is continued. Further, in a case where the predetermined analysis processing allowable condition is not satisfied, light emission is automatically switched to the mono-light emission from the multi-light emission. Accordingly, a mode is automatically switched to the mono-image display mode from the analysis processing mode.

After a mode is automatically switched to the mono-image display mode, the processor device 16 monitors whether or not the analysis processing restart condition is satisfied. In a case where the analysis processing restart condition is satisfied and the restart allowable condition is satisfied, light emission is automatically switched to the multi-light emission from the mono-light emission. Accordingly, a mode is switched to the analysis processing mode from the mono-image display mode. On the other hand, in a case where the restart allowable condition is not satisfied even though the analysis processing restart condition is satisfied, the automatic switching of light emission to the multi-light emission is prohibited. Further, even in a case where the analysis processing restart condition is not satisfied, the automatic switching of light emission to the multi-light emission is prohibited. In a case where the automatic switching of light emission to the multi-light emission is prohibited, the mono-image display mode is continued.

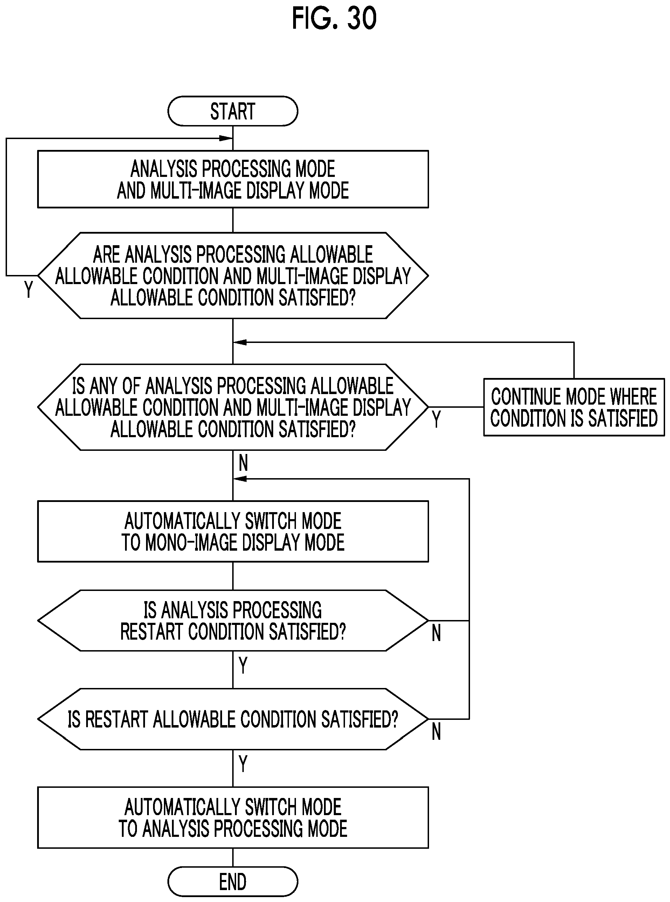

Next, a series of flows in the case of the switching pattern C (analysis processing mode: ON, multi-image display mode: ON) will be described with reference to a flowchart shown in Fig. 30. In a case where both the analysis processing mode and the multi-image display mode are activated, the first illumination light and the second illumination light are emitted while being switched according to a specific light emission pattern. Further, analysis processing is performed on the second image signals that are obtained from the image pickup of an object to be observed illuminated with the second illumination light. Furthermore, the analysis result of the analysis processing is superimposed and displayed on a display image based on the first image signals obtained from the image pickup of the object to be observed illuminated with the first illumination light, so that a display image with an analysis result is obtained. The display image with an analysis result is displayed on the display 18.

Further, the first observation image obtained from the image pickup of the object to be observed illuminated with the first illumination light and the second observation image obtained from the image pickup of the object to be observed illuminated with the second illumination light are displayed on the display 18 while being switched according to a specific display pattern (in this embodiment, at an interval of two frames).

As long as the analysis processing allowable condition and the multi-image display allowable condition are satisfied, the analysis processing mode and the multi-image display mode are continued. In a case where one of the analysis processing allowable condition and the multi-image display allowable condition is satisfied and the other thereof is not satisfied, the automatic switching of light emission to the mono-light emission from the multi-light emission is prohibited. In this case, a mode where a condition of the analysis processing allowable condition and the multi-image display allowable condition is satisfied is continued and a mode where the condition is not satisfied is stopped. In contrast, in a case where the analysis processing allowable condition is not satisfied and the multi-image display allowable condition is not satisfied, light emission is automatically switched to the mono-light emission from the multi-light emission. Accordingly, a mode is automatically switched to the mono-image display mode from the analysis processing mode and the multi-image display mode.

After a mode is automatically switched to the mono-image display mode, the processor device 16 monitors whether or not the analysis processing restart condition and the multi-image display restart condition are satisfied. In a case where the analysis processing restart condition is satisfied and the restart allowable condition is satisfied, light emission is automatically switched to the multi-light emission from the mono-light emission. Accordingly, a mode is automatically switched to the analysis processing mode. Further, in a case where the multi-image display restart condition is satisfied and the restart allowable condition is satisfied, light emission is automatically switched to the multi-light emission from the mono-light emission. Accordingly, a mode is automatically switched to the multi-image display mode.

In the embodiment, the first illumination light and the second illumination light have been emitted while being switched according to the specific light emission pattern, and the first observation image corresponding to the first illumination light and the second observation image corresponding to the second illumination light have been displayed on the display 18 while being switched according to the specific display pattern. However, three or more types of illumination light having wavelength ranges different from each other may be emitted while being switched according to a specific light emission pattern, and three or more types of observation images corresponding to the three or more types of illumination light may be displayed on the display 18 while being switched according to a specific display pattern.

The hardware structures of the processing units, which are included in the processor device 16 in the embodiment, such as the image acquisition unit 53, the DSP 56, the noise removing unit 58, the image processing unit 60, the parameter switching unit 62, the central controller 68, and the automatic mode switching unit 69, are various processors to be described below. The various processors include: a central processing unit (CPU) that is a general-purpose processor functioning as various processing units by executing software (program); a programmable logic device (PLD) that is a processor of which circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same type or different types of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

### Explanation of References

10: endoscope system
12: endoscope
12a: insertion part
12b: operation part
12c: bendable part
12d: distal end part
12e: angle knob
13a: mode changeover SWf
13b: static-image-acquisition instruction part
13c: zoom operation part
14: light source device
16: processor device
18: display
19: user interface
20: light source unit
20a: violet light emitting diode (V-LED)
20b: blue light emitting diode (B-LED)
20c: green light emitting diode (G-LED)
20d: red light emitting diode (R-LED)
21: light source processor
23: optical path-combination unit
30a: illumination optical system
30b: image pickup optical system
41: light guide
45: illumination lens
46: objective lens
47: zoom lens
47a: zoom drive unit
48: image pickup sensor
50: CDS/AGC circuit circuit
52: A/D converter
53: image acquisition unit
56: digital signal processor (DSP)
58: noise removing unit
60: image processing unit
62: parameter switching unit
63: static image storage unit
66: display controller
68: central controller
69: automatic mode switching unit
70: image-pickup-condition acquisition unit
72: observation portion acquisition section
74: brightness calculation section
76: magnification acquisition section
78: observation distance acquisition section
80: shake amount calculation section
82: multi-image display allowable condition-setting menu

## Claims

1. An endoscope system comprising:
a plurality of semiconductor light sources that emit light having wavelength ranges different from each other;
a light source processor that controls the plurality of semiconductor light sources and performs a control related to mono-light emission where only specific illumination light is emitted and a control related to multi-light emission where a plurality of types of illumination light, which include first illumination light and second illumination light having emission spectra different from each other, are emitted while being switched according to a specific light emission pattern;
an image pickup sensor that outputs a first image signal obtained from image pickup of an object to be observed illuminated with the first illumination light and a second image signal obtained from image pickup of the object to be observed illuminated with the second illumination light in a case of the multi-light emission; and
an image control processor,
wherein the image control processor
is capable of activating at least an analysis processing mode where a display image with an analysis result in which an analysis result obtained from analysis processing based on the second image signal is displayed on a display image based on the first image signal is displayed on a display,
is capable of activating a mono-image display mode where a control to display a specific observation image, which is obtained from image pickup of the object to be observed illuminated with the specific illumination light, on the display is performed, and
is capable of activating a multi-image display mode where a control to display a plurality of observation images, which include a first observation image based on the first image signal and a second observation image based on the second image signal, on the display while switching the plurality of observation images according to a specific display pattern is performed, and
the image control processor
automatically switches the mono-light emission and the multi-light emission on the basis of activation or non-activation of the analysis processing mode, activation or non-activation of the multi-image display mode, and a predetermined light source switching condition.

2. The endoscope system according to claim 1,
wherein in a case where the analysis processing mode is not activated and the multi-image display mode is activated,
the image control processor
automatically switches light emission to the multi-light emission from the mono-light emission during activation of the mono-light emission.

3. The endoscope system according to claim 2,
wherein in a case where light emission is automatically switched to the mono-light emission from the multi-light emission and a mode is automatically switched to the mono-image display mode from the multi-image display mode since a multi-image display allowable condition included in the light source switching condition is not satisfied,
the image control processor
automatically switches light emission to the multi-light emission from the mono-light emission and activates the multi-image display mode in a case where a multi-image display restart condition is satisfied and a restart allowable condition is satisfied in the light source switching condition and prohibits light emission from being automatically switched to the multi-light emission in a case where the multi-image display restart condition is satisfied and the restart allowable condition is not satisfied.

4. The endoscope system according to claim 3,
wherein the restart allowable condition is a case where a user does not select the mono-light emission.

5. The endoscope system according to claim 3 or 4,
wherein the restart allowable condition is a case where a restart allowable time does not elapse yet after light emission is switched to the mono-light emission.

6. The endoscope system according to any one of claims 3 to 5,
wherein the restart allowable condition is a case where the multi-light emission is implementable.

7. The endoscope system according to any one of claims 3 to 6, further comprising:
a magnification change unit that is used to change a magnification of the object to be observed,
wherein the restart allowable condition is a case where use or non-use of the magnification change unit is not switched.

8. The endoscope system according to any one of claims 3 to 7,
wherein the restart allowable condition is capable of being set by a user.

9. The endoscope system according to any one of claims 3 to 8,
wherein the multi-image display restart condition is that a change in an image pickup condition about the object to be observed is within a multi-light emission allowable range.

10. The endoscope system according to claim 1,
wherein in a case where the analysis processing mode is activated and the multi-image display mode is not activated,
the image control processor
automatically switches light emission to the multi-light emission from the mono-light emission according to start of the activation of the analysis processing mode during activation of the mono-light emission.

11. The endoscope system according to claim 10,
wherein in a case where light emission is automatically switched to the mono-light emission from the multi-light emission and a mode is automatically switched to the mono-image display mode from the analysis processing mode since an analysis processing allowable condition included in the light source switching condition is not satisfied,
the image control processor
automatically switches light emission to the multi-light emission from the mono-light emission and activates the analysis processing mode in a case where an analysis processing restart condition is satisfied and a restart allowable condition is satisfied in the light source switching condition and
prohibits light emission from being automatically switched to the multi-light emission in a case where the analysis processing restart condition is satisfied and the restart allowable condition is not satisfied.

12. The endoscope system according to claim 1,
wherein in a case where the analysis processing mode is activated and the multi-image display mode is activated,
the image control processor
automatically switches light emission to the multi-light emission from the mono-light emission according to start of the activation of the analysis processing mode and the multi-image display mode during activation of the mono-light emission,
prohibits light emission from being automatically switched to the mono-light emission in a case where any of an analysis processing allowable condition and a multi-image display allowable condition included in the light source switching condition is satisfied during activation of the multi-light emission, and
automatically switches light emission to the mono-light emission from the multi-light emission in a case where the analysis processing allowable condition is not satisfied and the multi-image display allowable condition is not satisfied during activation of the multi-light emission.

13. A method of operating an endoscope system including
a plurality of semiconductor light sources that emit light having wavelength ranges different from each other,
a light source processor that controls the plurality of semiconductor light sources and performs a control related to mono-light emission where only specific illumination light is emitted and a control related to multi-light emission where a plurality of types of illumination light, which include first illumination light and second illumination light having emission spectra different from each other, are emitted while being switched according to a specific light emission pattern,
an image pickup sensor that outputs a first image signal obtained from image pickup of an object to be observed illuminated with the first illumination light and a second image signal obtained from image pickup of the object to be observed illuminated with the second illumination light in a case of the multi-light emission, and
an image control processor,
wherein the image control processor
is capable of activating an analysis processing mode where a display image with an analysis result in which an analysis result obtained from analysis processing based on the second image signal is displayed on a display image based on the first image signal is displayed on a display,
is capable of activating a mono-image display mode where a control to display a specific observation image, which is obtained from image pickup of the object to be observed illuminated with the specific illumination light, on the display is performed, and
is capable of activating a multi-image display mode where a control to display a plurality of observation images, which include a first observation image based on the first image signal and a second observation image based on the second image signal, on the display while switching the plurality of observation images according to a specific display pattern is performed, and
the image control processor
automatically switches the mono-light emission and the multi-light emission on the basis of activation or non-activation of the analysis processing mode, activation or non-activation of the multi-image display mode, and a predetermined light source switching condition.
